(19) Europäisches Patentamt
European Patent Office
Office européen des brevets

(11) **EP 3 765 107 B1**

(12) **EUROPEAN PATENT SPECIFICATION**

(45) Date of publication and mention
of the grant of the patent:
**21.05.2025 Bulletin 2025/21**

(21) Application number: **18792579.7**

(22) Date of filing: **14.09.2018**

(51) International Patent Classification (IPC):
***A61M 1/00*** (2006.01)

(52) Cooperative Patent Classification (CPC):
**A61M 1/76; A61M 1/84; A61M 1/86;** A61M 2206/20

(86) International application number:
**PCT/US2018/051086**

(87) International publication number:
**WO 2019/177661 (19.09.2019 Gazette 2019/38)**

(54) **SURGICAL SUCTION SYSTEM**

CHIRURGISCHES ABSAUGESYSTEM

SYSTEME CHIRURGICAL D'ASPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.03.2018 US 201862642693 P**

(43) Date of publication of application:
**20.01.2021 Bulletin 2021/03**

(60) Divisional application:
**25166428.0**

(73) Proprietor: **CONMED Corporation**
**Largo, FL 33773 (US)**

(72) Inventor: **SNYDER, Jessica**
**Poland, NY 13431 (US)**

(74) Representative: **Strehl & Partner mbB**
**Maximilianstrasse 54**
**80538 München (DE)**

(56) References cited:
**US-A- 4 455 140      US-A- 5 665 080**
**US-A- 5 921 970**

## Description

CROSS-REFERENCE TO RELATED APPLICATIONS

BACKGROUND OF THE INVENTION

### 1. Field of the Invention

The present invention is directed generally to medical devices used during surgical procedures and, more particularly, to a surgical suction system with adapters for interchanging instruments and connecting to a variety of vacuum systems and collection devices.

### 2. Description of Related Art

During the performance of various medical procedures it frequently becomes necessary to remove material such as blood and bone chips during an orthopedic procedure, by suctioning. Conventional suction canisters connected to the suction instruments used in medical procedures have standard fittings for receiving suction tubing. Conventional suction tubing has an inner diameter typically within the range of 0.32 to 0.71 cm ( $\frac{1}{8}$ to $\frac{9}{32}$ inch). However, in many orthopedic procedures, the nature of the materials being removed by the suctioning are such that the materials clog the standard tubing, instruments, and fittings used for connecting suction tubing.

In a single standard hip or knee replacement (or revision) procedure, a surgeon may unclog the suction tubing up to 15 times. As mentioned above, the suction tubing can become clogged with a mixture of solid and liquid biological materials, such as blood, fat, bone chips, and coagulated blood. To unclog the suction system, the surgeon has to disassemble and reassemble the components of the system. Thus, unclogging the suction system is labor intensive and time consuming.

In some cases, the surgical procedure may require use of a small suction instrument. For example, a small suction instrument may be required for small incisions made at the beginning of a surgical procedure when the surgical site is small and the suction is used for liquid biological materials only. Currently, small and large suction instruments are components of their own separate suction systems. In other words, a surgeon must switch suction systems to use a different sized suction instrument.

Accordingly, there is a need for a suction system that minimizes clogging and allows the interchangeable use of small and large suction instruments. US 5 921 970 A discloses a suction system with the features in the preamble of present claim 1. Other conventional suction systems are described in US 5 665 080 A and US 4 455 140 A.

SUMMARY OF THE INVENTION

The present invention as defined in the appended claims is directed to a surgical suction system with adapters for interchanging instruments and connecting to a variety of vacuum systems and collection devices. According to one aspect, the suction system includes a Yankauer suction device having a distal tip with a first diameter, and a proximal end with exterior hose barbs (or, alternatively, threads) and a second diameter. The second diameter is greater than the first diameter. The suction system also includes cannulated tubing having a distal end and a proximal end. The distal end of the cannulated tubing is configured to mate with the exterior hose barbs of the Yankauer suction device. The suction system further includes an adapter having a distal end with exterior hose barbs and a proximal end. The exterior hose barbs of the adapter are configured to mate with the proximal end of the cannulated tubing.

According to another aspect, the suction system includes a Yankauer suction device having a distal tip with a first diameter, and a proximal end with exterior hose barbs and a second diameter. The second diameter is greater than the first diameter. The suction system also includes cannulated tubing having a distal end and a proximal end. The distal end of the cannulated tubing is configured to mate with the exterior hose barbs of the Yankauer suction device. The suction system further includes a first adapter having a distal end with exterior hose barbs and a proximal end. The exterior hose barbs of the adapter are configured to mate with the proximal end of the cannulated tubing. The suction system also includes a second instrument having a proximal end, which is configured to mate with a proximal end of a second adapter. The second adapter has a proximal end with exterior hose barbs, which are configured to mate with the distal end of the cannulated tubing. The Yankauer suction device and the second instrument are interchangeable. In a first configuration, the proximal end of the Yankauer suction device is connected to the distal end of the cannulated tubing. In a second configuration, the proximal end of the second instrument is connected to the distal end of the second adapter and the proximal end of the second adapter is connected to the distal end of the cannulated tubing.

It should be appreciated that all combinations of the foregoing concepts and additional concepts discussed in greater detail below (provided such concepts are not mutually inconsistent) are contemplated as being part of the inventive subject matter disclosed herein.

These and other aspects of the invention will be apparent from and elucidated with reference to the embodiment(s) described hereinafter. The invention is defined by the appended claims.

BRIEF DESCRIPTION OF THE DRAWINGS

One or more aspects of the present invention are particularly pointed out and distinctly claimed as examples in the claims at the conclusion of the specification. The foregoing and other objects, features, and advantages of the invention are apparent from the following description taken in conjunction with the accompanying drawings in which:

FIG. 1 is an exploded side view schematic representation of the suction system, according to an embodiment;

FIG. 2 is a front (distal) view schematic representation of the large bore Yankauer suction device, according to an embodiment.

FIG. 3 is a side interior view schematic representation of the suction system, according to an embodiment.

FIG. 4 is a perspective side view schematic representation of the flexible adapter, according to an embodiment.

FIG. 5 is a back (proximal) perspective view schematic representation of the flexible adapter, according to embodiment.

FIG. 6 is a side view schematic representation of the flexible adapter, according to an embodiment;

FIG. 7 is a cross-sectional view schematic representation of the flexible adapter of FIG. 6; and

FIG. 8 is a perspective view schematic representation of a pour spout adapter, according to an embodiment.

## DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. The invention is defined by the appended claims.

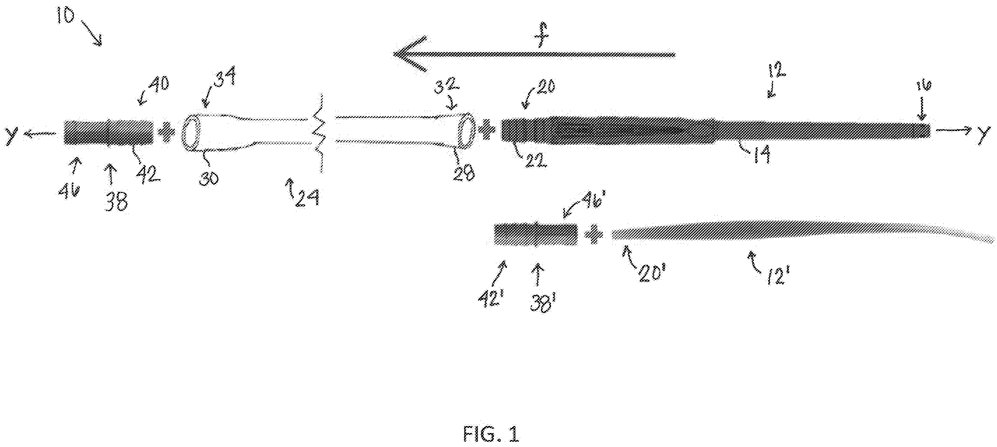

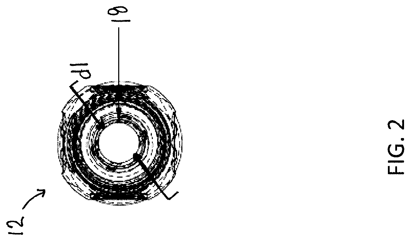

Referring now to the figures, wherein like reference numerals refer to like parts throughout, FIG. 1 shows an exploded side view schematic representation of the suction system 10, according to an embodiment. The suction system 10 comprises a large bore suction instrument 12, such as a Yankauer suction tool/device (hereinafter "large bore Yankauer"). The large bore Yankauer 12 comprises a body 14 with a distal tip 16 having a bore 18 extending therethrough, as shown in FIG. 2. In the depicted embodiment, the bore 18 is relatively large in order to remove both liquid and solid biological materials (e.g., blood, fat, bone chips) from the surgical site. In one embodiment, the bore 18 has an inner diameter $d1$ of 6.00 mm; however, a bore 18 of an alternative size may be used as long as the inner diameter is sufficiently large to allow for the passage of both liquid and solid biological materials. Specifically, as used herein "large bore" is defined to include a broad range of bore sizes having a diameter within the range of 5-10 mm, with the inner diameter $d1$ of the bore 18 preferably within the range of 6-8 mm.

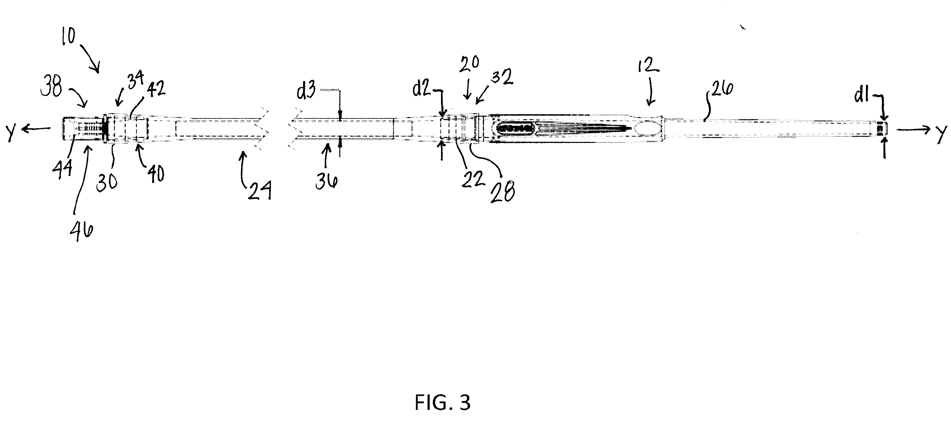

Referring back to FIG. 1, the body 14 of the large bore Yankauer 12 extends from its distal tip 16 to a proximal end 20 along a central longitudinal y - y axis. The proximal end 20 of the large bore Yankauer 12 comprises exterior hose barbs 22. Hose barbs 22 are understood by one of ordinary skill in the art as barb-like rings which allow for each push-connection and prevent easy disconnection to tubing. The exterior hose barbs 22 at the proximal end 20 are sized and configured to removably attach to cannulated flexible tubing 24, as shown in FIG. 3. As also shown in FIG. 3, a channel 26 extends along the central longitudinal y - y axis through the body 14 of the large bore Yankauer 12 from the bore 18 at the distal tip 16 to the proximal end 20. In one embodiment, the inner diameter of the channel 26 is smallest at the distal tip 16 (i.e., $d1$ of bore 18 in FIG. 2). For example, in one embodiment, the inner diameter of the channel 26 increases from the inner diameter $d1$ of the distal tip 16 to the inner diameter $d2$ (FIG. 3) at the proximal end 20. Increasing the inner diameter of the large bore Yankauer 12 from distal tip 16 to the proximal end 20 with the direction of suction $f$ (FIG. 1) provides extra space for flowing biological materials and minimizes clogs.

As stated above, the proximal end 20 of the large bore Yankauer 12 is sized and configured to mate with or otherwise connect to the cannulated flexible tubing 24. In the embodiment shown in FIG. 1, the cannulated flexible tubing 24 comprises a pair of flared ends 28, 30, one at a distal end 32 of the flexible tubing 24 and one at a proximal end 34 of the flexible tubing 24, respectively. The flared ends 28, 30 are portions of the flexible tubing 24 wherein the flexible tubing 24 is tapered or otherwise has a changing inner diameter. The flared ends 28, 30 accommodate connections between the flexible tubing 24 and instruments and/or connectors, and are configured and/or structured to prevent pinching or kinking of the tube adjacent to the flared end (conventional tubing without the flared ends kink or pinch at a section adjacent the connected ends of the tube). Accordingly, other dimensions and configurations of the flexible tubing 24 may be used as long as the distal and proximal ends 32, 34 are suitable for stable connection to the instruments and/or connectors.

In the embodiment depicted in FIG. 1, an inner diameter of the cannulated flexible tubing 24 increases toward the distal end 32, creating the first flared end 28 and increases in the opposite direction toward the proximal end 34, creating the second flared end 30. As the inner diameter of the cannulated flexible tubing 24 increases toward both the distal and proximal ends 32, 34, the inner diameter of the flexible tubing 24 is smallest at a central portion 36 of the flexible tubing 24 between the distal and proximal ends 32, 34, as shown in FIG. 3. In FIG. 3, the central portion 36 extends to distal and proximal ends 32, 34 and has an inner diameter $d3$, which is greater than the inner diameter $d1$ of the bore 18 at the distal tip 16 of the large bore Yankauer 12. For example, in one embodiment, the inner diameter $d1$ of the first bore is 6.00 mm and the inner diameter $d3$ of the central portion 36 of the flexible tubing 24 is 8.38 mm. In an alternative embodiment, $d3 > d2 > d1$ such that the inner diameter of the suction system 10 increase with the direction of suction $f$ (FIG. 1). In another embodiment, the inner diameter $d3$ of the central portion 36 is 11/32 inch, which is larger than the inner diameter of tubing generally used with Yankauer suction devices. A relatively large inner diameter of the flexible tubing 24 increases flow in the direction of suction $f$ (FIG. 1), which minimizes clogging.

Still referring to FIG. 3, the first flared end 28 of the cannulated flexible tubing 24 is sized and configured to removably receive the proximal end 20 of the large bore Yankauer 12. In particular, FIG. 3 shows that the first flared end 28 engages or catches on each of the barbs 22 on the proximal end 20 of the large bore Yankauer 12. The second flared end 30 of the cannulated flexible tubing 24 is sized and configured to removably receive a flexible adapter 38 (also in FIG. 1). As shown in FIG. 1, the flexible adapter 38 comprises a distal end 40 with exterior hose barbs 42. The exterior hose barbs 42 are sized and configured to mate with or otherwise connect to the second flared end 30 of the cannulated flexible tubing 24, as shown in FIG. 3. In the depicted embodiment, the second flared end 30 engages or catches on each of the barbs 42 on the distal end 40 of the flexible adapter 38.

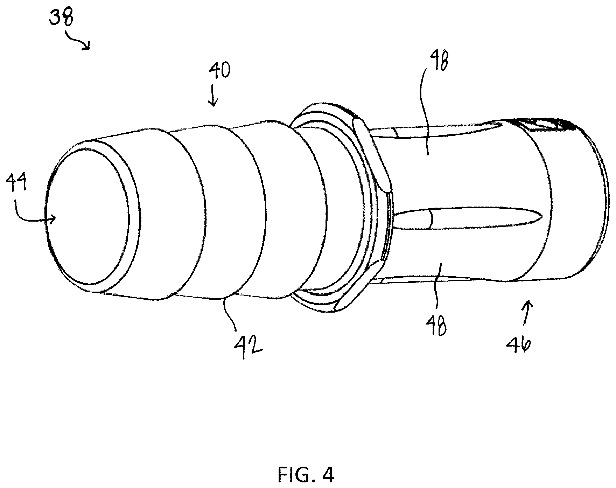

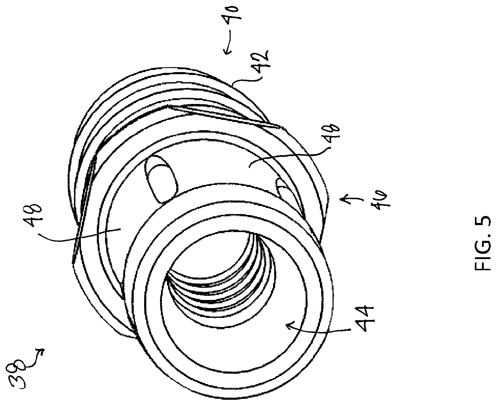

Referring now to FIGs. 4-5, there are shown various views schematic representations of the flexible adapter 38, according to an embodiment. FIG. 4 shows a perspective side view of the flexible adapter 38. In the depicted embodiment, the flexible adapter 38 has exterior hose barbs 42 on its distal end 40, as described above. In one embodiment, the exterior hose barbs 42 of the flexible adapter 38 match or are substantially similar to the exterior hose barbs 22 at the proximal end 20 of the large bore Yankauer 12. FIG. 5 shows a perspective back (proximal) view schematic representation of the flexible adapter 38. FIGs. 4 and 5 show a bore 44 extending from a proximal end 46 to the distal end 40 of the flexible adapter 38. FIG. 3 also shows the bore 44 extending therethrough. When assembled, the suction system 10 comprises a pathway for biological materials from the distal tip 16 of the large bore Yankauer 12 to a proximal end 46 of the flexible adapter 38. The pathway includes the bore 18 and channel 26 in the large bore Yankauer 12, the cannulated flexible tubing 24, and the bore 44 of the flexible adapter 38, as shown in FIG. 3.

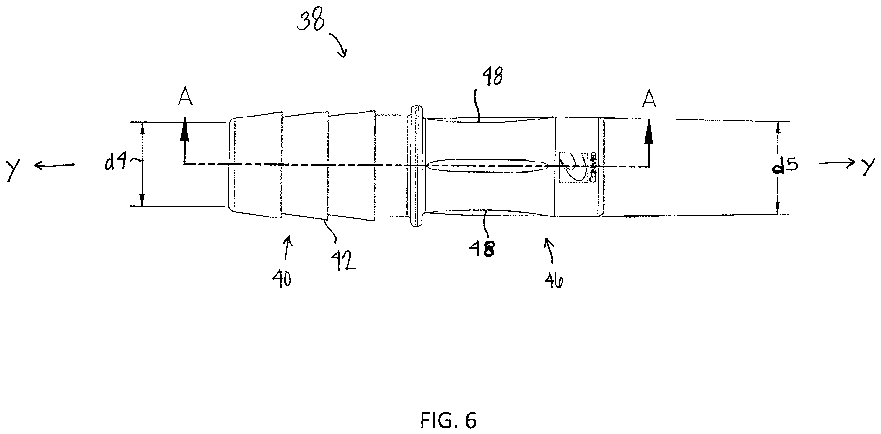

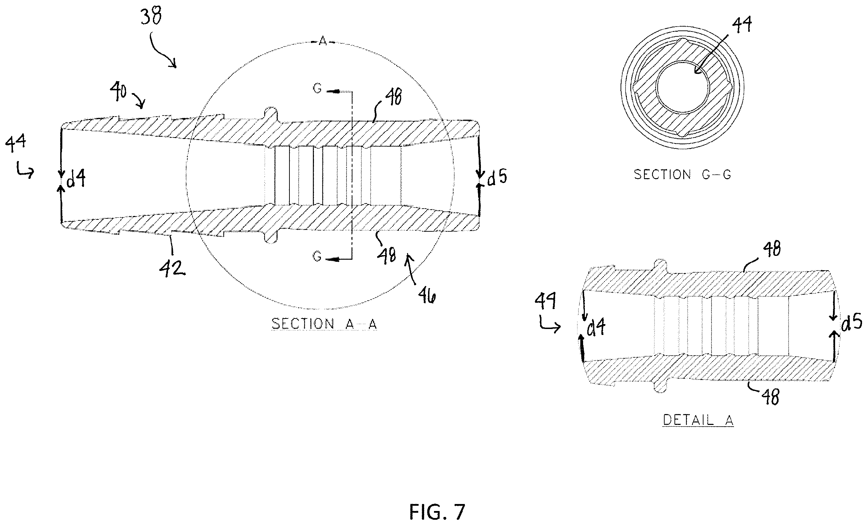

FIGs. 6-7 also show various views schematic representations of the flexible adapter 38, according to an embodiment. FIG. 6 shows a side view schematic representation of the flexible adapter 38, while FIG. 7 shows a cross-sectional view schematic representation of the flexible adapter 38 of FIG. 6. In the depicted embodiment, as described above, the bore 44 extends from the proximal end 46 to the distal end 40 of the flexible adapter 38. In one embodiment, the smallest inner diameter of the flexible adapter 38 is larger than the inner diameter $d1$ of the bore 18 of the distal tip 16 of the large bore Yankauer 12. For example, the smallest inner diameter of the flexible adapter 38 is 7.24 mm, while the inner diameter $d1$ of the bore 18 of the distal tip 16 of the large bore Yankauer 12 is 6.00 mm.

In the present invention, the inner diameter $d4$ at the distal end 40 of the flexible adapter 38 is smaller than the inner diameter $d5$ at the proximal end 46 of the flexible adapter 38. The largest inner diameter of the suction system 10 is the inner diameter $d5$ at the proximal end 46 of the flexible adapter 38 such that the inner diameter of the suction system 10 increases from the distal tip 16 of the large bore Yankauer 12 to the proximal end 46 of the flexible adapter 38. An increasing inner diameter of the suction system 10 in the direction of suction $f$ (FIG. 1) allows for biological materials of varying sizes and consistencies to pass through the suction system 10, increasing flow to the proximal end 46 and minimizing clogs.

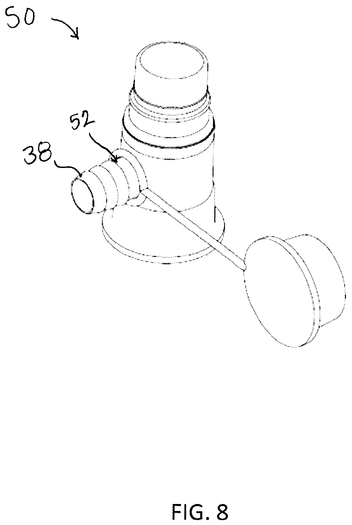

Referring back to FIG. 4, the proximal end 46 of the flexible adapter 38 is configured to interface the cannulated flexible tubing 24 with a suction collection canister, other container, or existing instruments having a relatively small diameter (as described in detail below, and as should be understood by a person of ordinary skill in the art in conjunction with a review of this disclosure). For example, the proximal end 46 of the flexible adapter 38 can be connected to a port on a collection canister, such as a collection canister on a cart (e.g., portable vacuum system). In another example, the cannulated flexible tubing 24 is configured to interface with a flexible adapter 38 which is fixed (permanently or removably) to a port 52 on a pour spout adapter 50 for a collection canister, as shown in FIG. 8. As understood by one of ordinary skill in the art, the pour spout adapter 50 connects to a suction collection canister (not shown) so that biological material removed by the suction system 10 can be poured from the collection canister without removal of its lid (or other covering). In another embodiment, the proximal end 46 of the flexible adapter 38 is configured to connect to a port for a wall vacuum. The proximal end 46 of the flexible adapter 38 can be connected to a port in any of the examples described above as understood by one of ordinary skill in the art.

As shown in FIG. 4-7, the proximal end 46 of the flexible adapter 38 has one or more flats 48. In the depicted embodiment, the flats 48 are spaced and extend along the length of the flexible adapter 38. The flats 48 are configured and/or structured to prevent the flexible adapter 38 from rolling off the instrument table or other surface when the flexible adapter 38 (or suction system 10) is not in use. In an embodiment, the flats 48 extend in a direction substantially parallel to the central longitudinal axis y - y extending through the flexible adapter 38.

Turning back to FIG. 1, an additional flexible adapter 38' can be used to connect a small bore Yankauer 12' with the first flared end 28 (i.e., distal end 32) of the cannulated flexible tubing 24. As mentioned above, the flexible adapter 38' can be used to connect the flexible tubing 24 to a smaller instrument (relative to the large bore Yankauer 12). It is important to note that when the additional flexible adapter 38' is used, it is the same as the flexible adapter shown in FIGs. 4-7; however, the distal end 40 of the flexible adapter 38 is the same as the proximal end 40' of the additional flexible adapter 38' and the proximal end 46 of the flexible adapter 38 is the same as the distal end 46' of the additional flexible adapter 38'. In other words, in one embodiment, the additional flexible adapter 38' is a rotated configuration (180°) of the flexible adapter 38 in FIGs. 4-7.

As shown in FIG. 1, the distal end 46' of the flexible adapter 38' connects to a proximal end 20' of the small bore Yankauer 12' (or other small instrument) and the proximal end 42' of the flexible adapter 38' connects to the first flared end 28 of the flexible tubing 24. Thus, the flexible adapter 38' and connected small bore Yankauer 12' can be interchanged with the large bore Yankauer 12, as shown in FIG. 1. The small bore Yankauer 12' can be used to suction or remove liquids from surgical areas, such as small incisions. The ability to quickly interchange the large bore Yankauer 12 with a smaller instrument 12' allows the user to select the instrument (e.g., large bore Yankauer 12 or small bore Yankauer 12') based on factors, such as the size of the surgical site and the biological materials that are being removed from the surgical site.

The invention is defined by the appended claims.

**Claims**

1. A suction system (10), comprising:

   a Yankauer suction device (12) having a distal tip (16) with a first diameter (d1) and a proximal end (20), the proximal end (20) having a second diameter (d2), wherein the second diameter (d2) is greater than the first diameter (d1);
   cannulated flexible tubing (24) having a distal end (32) and a proximal end (34), the distal end (32) configured to mate with the proximal end (20) of the Yankauer suction device (12);
   a first adapter (38) having a distal end (40) and a proximal end (46), the distal end (40) configured to mate with the proximal end (34) of the cannulated tubing (24); and
   a channel (26) extending from the distal tip (16) of the Yankauer suction device (12) to the proximal end (20) of the Yankauer suction device (12),
   wherein the channel (26) has an inner diameter increasing with the direction of suction (f) from the first diameter (d1) of the distal tip (16) to the second diameter (d2) of the proximal end (20), **characterized in that** an inner diameter (d4) at the distal end (40) of the first adapter (38) is smaller than an inner diameter (d5) at the proximal end (46) of the first adapter (38), and the inner diameter (d5) at the proximal end (46) of the first adapter (38) is the largest inner diameter of the suction system (10).

2. The suction system (10) of claim 1, wherein the channel (26) extends from the distal tip (16) of the Yankauer suction device (12) to the proximal end (46) of the first adapter (38), and the channel (26) has an inner diameter increasing from the distal tip (16) of the Yankauer suction device (12) to the proximal end (46) of the first adapter (38).

3. The suction system (10) of claim 1, wherein the cannulated tubing (24) has a third diameter (d3) greater than the first diameter (d1).

4. The suction system (10) of claim 1, wherein the distal end (32) of the cannulated tubing (24) is a first flared end (28) and the proximal end (34) of the cannulated tubing (24) is a second flared end (30).

5. The suction system (10) of claim 4, wherein an inner diameter of the cannulated tubing (24) increases from a central position (36) toward the first flared end (28) and toward the second flared end (30).

6. The suction system (10) of claim 1, wherein the distal end (40) of the first adapter (38) has a fourth diameter (d4), which is greater than the first diameter (d1) of the distal tip (16) of the Yankauer suction device (12).

7. The suction system (10) of claim 6, wherein the proximal end (46) of the first adapter (38) has a fifth diameter (d5), which is greater than the first diameter (d1) of the distal tip (16) of the Yankauer suction device (12).

8. The suction system (10) of claim 7, wherein the fifth diameter (d5) is greater than the fourth diameter (d4).

9. The suction system (10) of claim 1, further comprising one or more flats (48) extending along the length of the proximal end (46) of the first adapter (38).

10. The suction system (10) of claim 1, wherein

    the distal end (40) of the first adapter (38) includes exterior hose barbs (42) and the proximal end (20) of the Yankauer suction device (12) has exterior hose barbs (22), and

the exterior hose barbs (42) of the first adapter (38) match the exterior hose barbs (22) on the proximal end (20) of the Yankauer suction device (12).

11. The suction system (10) of claim 1, further comprising:

a second instrument (12') having a proximal end (20') configured to mate with a distal end (46') of a second adapter (38'), the second adapter (38') having a proximal end (42') configured to mate with the distal end (32) of the cannulated tubing (24);

wherein the Yankauer suction device (12) and the second instrument (12') are interchangeable such that in a first configuration, the proximal end (20) of the Yankauer suction device (12) is connected to the distal end (32) of the cannulated tubing (24), and in a second configuration, the proximal end (42') of the second instrument (12') is connected to the distal end (46') of the second adapter (38'), which is connected to the distal end (32) of the cannulated tubing (24).

12. The suction system (10) of claim 11, wherein the second instrument (12') is a small bore Yankauer suction device that has a smaller inner diameter than the inner diameter (d1) of the Yankauer suction device (12).

13. The suction system (10) of claim 11, wherein in the first configuration, the direction of suction (f) is from the distal tip (16) of the Yankauer suction device (12) to the proximal end (46) of the first adapter (38).

14. The suction system (10) of claim 11, wherein in the second configuration, the direction of suction (f) is from the second instrument (12') to the proximal end (46) of the first adapter (38).

15. The suction system (10) of claim 11, wherein

the distal end (40) of the first adapter (38), the distal end (46') of the second adapter (38'), and the proximal end (20) of the Yankauer suction device (12) each include exterior hose barbs (22, 42), and

the exterior hose barbs (42) of the first adapter (38) match the exterior hose barbs on the second adapter (38') and the exterior hose barbs (22) on the proximal end (20) of the Yankauer suction device (12).

**Patentansprüche**

1. Saugsystem (10), umfassend:

eine Yankauer-Saugvorrichtung (12) mit einer distalen Spitze (16) mit einem ersten Durchmesser (d1) und einem proximalen Ende (20), wobei das proximale Ende (20) einen zweiten Durchmesser (d2) aufweist, wobei der zweite Durchmesser (d2) größer ist als der erste Durchmesser (d1);

einen kanülierten flexiblen Schlauch (24) mit einem distalen Ende (32) und einem proximalen Ende (34), wobei das distale Ende (32) dazu konfiguriert ist, mit dem proximalen Ende (20) der Yankauer-Saugvorrichtung (12) zusammenzupassen;

einen ersten Adapter (38) mit einem distalen Ende (40) und einem proximalen Ende (46), wobei das distale Ende (40) dazu konfiguriert ist, mit dem proximalen Ende (34) des kanülierten Schlauchs (24) zusammenzupassen; und

einen Kanal (26), der sich von der distalen Spitze (16) der Yankauer-Saugvorrichtung (12) zu dem proximalen Ende (20) der Yankauer-Saugvorrichtung (12) erstreckt,

wobei der Kanal (26) einen Innendurchmesser aufweist, der in der Ansaugrichtung (f) von dem ersten Durchmesser (d1) der distalen Spitze (16) zu dem zweiten Durchmesser (d2) des proximalen Endes (20) zunimmt,

**dadurch gekennzeichnet, dass** ein Innendurchmesser (d4) an dem distalen Ende (40) des ersten Adapters (38) kleiner ist als ein Innendurchmesser (d5) an dem proximalen Ende (46) des ersten Adapters (38), und der Innendurchmesser (d5) an dem proximalen Ende (46) des ersten Adapters (38) der größte Innendurchmesser des Saugsystems (10) ist.

2. Saugsystem (10) nach Anspruch 1, wobei sich der Kanal (26) von der distalen Spitze (16) der Yankauer-Saugvorrichtung (12) zu dem proximalen Ende (46) des ersten Adapters (38) erstreckt und der Kanal (26) einen Innendurchmesser aufweist, der von der distalen Spitze (16) der Yankauer-Saugvorrichtung (12) zu dem proximalen Ende (46) des ersten Adapters (38) zunimmt.

3. Saugsystem (10) nach Anspruch 1, wobei der kanülierte Schlauch (24) einen dritten Durchmesser (d3) hat, der größer ist als der erste Durchmesser (d1).

4. Saugsystem (10) nach Anspruch 1, wobei das distale Ende (32) des kanülierten Schlauchs (24) ein erstes aufgeweitetes Ende (28) und das proximale Ende (34) des kanülierten Schlauchs (24) ein zweites aufgeweitetes Ende (30) ist.

**5.** Saugsystem (10) nach Anspruch 4, wobei ein Innendurchmesser des kanülierten Schlauchs (24) von einer zentralen Position (36) zu dem ersten aufgeweiteten Ende (28) und zu dem zweiten aufgeweiteten Ende (30) hin zunimmt.

**6.** Saugsystem (10) nach Anspruch 1, wobei das distale Ende (40) des ersten Adapters (38) einen vierten Durchmesser (d4) aufweist, der größer ist als der erste Durchmesser (d1) der distalen Spitze (16) der Yankauer-Saugvorrichtung (12).

**7.** Saugsystem (10) nach Anspruch 6, wobei das proximale Ende (46) des ersten Adapters (38) einen fünften Durchmesser (d5) aufweist, der größer ist als der erste Durchmesser (d1) der distalen Spitze (16) der Yankauer-Saugvorrichtung (12).

**8.** Saugsystem (10) nach Anspruch 7, wobei der fünfte Durchmesser (d5) größer ist als der vierte Durchmesser (d4).

**9.** Saugsystem (10) nach Anspruch 1, ferner umfassend eine oder mehrere Abflachungen (48), die sich entlang der Länge des proximalen Endes (46) des ersten Adapters (38) erstrecken.

**10.** Saugsystem (10) nach Anspruch 1, wobei

das distale Ende (40) des ersten Adapters (38) äußere Schlauchvorsprünge (42) und das proximale Ende (20) der Yankauer-Saugvorrichtung (12) äußere Schlauchvorsprünge (22) aufweist, und
die äußeren Schlauchvorsprünge (42) des ersten Adapters (38) mit den äußeren Schlauchvorsprünge (22) am proximalen Ende (20) der Yankauer-Saugvorrichtung (12) zusammenpassen.

**11.** Saugsystem (10) nach Anspruch 1, ferner umfassend:

ein zweites Instrument (12') mit einem proximalen Ende (20'), das dazu ausgelegt ist, mit einem distalen Ende (46') eines zweiten Adapters (38') zusammenzupassen, wobei der zweite Adapter (38') ein proximales Ende (42') aufweist, das dazu konfiguriert ist, mit dem distalen Ende (32) des kanülierten Schlauchs (24) zusammenzupassen;
wobei die Yankauer-Saugvorrichtung (12) und das zweite Instrument (12') so austauschbar sind, dass in einer ersten Konfiguration das proximale Ende (20) der Yankauer-Saugvorrichtung (12) mit dem distalen Ende (32) des kanülierten Schlauchs (24) verbunden ist, und in einer zweiten Konfiguration das proximale Ende (42') des zweiten Instruments (12') mit dem distalen Ende (46') des zweiten Adapters (38') verbunden ist, der mit dem distalen Ende (32) des kanülierten Schlauchs (24) verbunden ist.

**12.** Saugsystem (10) nach Anspruch 11, wobei das zweite Instrument (12') eine Yankauer-Saugvorrichtung mit kleiner Bohrung ist, die einen kleineren Innendurchmesser als den Innendurchmesser (d1) der Yankauer-Saugvorrichtung (12) aufweist.

**13.** Saugsystem (10) nach Anspruch 11, wobei in der ersten Konfiguration die Saugrichtung (f) von der distalen Spitze (16) der Yankauer-Saugvorrichtung (12) zum proximalen Ende (46) des ersten Adapters (38) verläuft.

**14.** Saugsystem (10) nach Anspruch 11, wobei in der zweiten Konfiguration die Saugrichtung (f) vom zweiten Instrument (12') zum proximalen Ende (46) des ersten Adapters (38) verläuft.

**15.** Saugsystem (10) nach Anspruch 11, wobei

das distale Ende (40) des ersten Adapters (38), das distale Ende (46') des zweiten Adapters (38') und das proximale Ende (20) der Yankauer-Saugvorrichtung (12) jeweils äußere Schlauchanschlüsse (22, 42) aufweisen, und
die äußeren Schlauchvorsprünge (42) des ersten Adapters (38) mit den äußeren Schlauchvorsprüngen am zweiten Adapter (38') und den äußeren Schlauchvorsprüngen (22) am proximalen Ende (20) der Yankauer-Saugvorrichtung (12) zusammenpassen.

**Revendications**

**1.** Système (10) d'aspiration, comprenant,

un dispositif (12) d'aspiration de Yankauer ayant un embout distal (16) avec un premier diamètre (d1) et une extrémité proximale (20), l'extrémité proximale (20) ayant un deuxième diamètre (d2), dans lequel le deuxième diamètre (d2) est plus grand que le premier diamètre (d1) ;
un tube flexible canulé (24) ayant une extrémité distale (32) et une extrémité proximale (34), l'extrémité distale (32) configurée pour se coupler avec l'extrémité proximale (20) du dispositif (12) d'aspiration de Yankauer ;
un premier adaptateur (38) ayant une extrémité distale (40) et une extrémité proximale (46), l'extrémité distale (40) configurée pour se coupler avec l'extrémité proximale (34) du tube canulé (24) ; et
un canal (26) s'étendant depuis l'embout distal (16) du dispositif (12) d'aspiration de Yankauer jusqu'à l'extrémité proximale (20) du dispositif (12) d'aspiration de Yankauer,

dans lequel le canal (26) a un diamètre intérieur s'accroissant avec le sens d'aspiration (f) du premier diamètre (d1) de l'embout distal (16) jusqu'au deuxième diamètre (d2) de l'extrémité proximale (20),

**caractérisé en ce qu'**un diamètre intérieur (d4) à l'extrémité distale (40) du premier adaptateur (38) est plus petit qu'un diamètre intérieur (d5) à l'extrémité proximale (46) du premier adaptateur (38), et le diamètre intérieur (d5) à l'extrémité proximale (46) du premier adaptateur (38) est le diamètre intérieur le plus grand du système (10) d'aspiration.

2. Système (10) d'aspiration selon la revendication 1, dans lequel le canal (26) s'étend depuis l'embout distal (16) du dispositif (12) d'aspiration de Yankauer jusqu'à l'extrémité proximale (46) du premier adaptateur (38), et le canal (26) a un diamètre intérieur s'accroissant depuis l'embout distal (16) du dispositif (12) d'aspiration de Yankauer jusqu'à l'extrémité proximale (46) du premier adaptateur (38).

3. Système (10) d'aspiration selon la revendication 1, dans lequel le tube canulé (24) a un troisième diamètre (d3) plus grand que le premier diamètre (d1).

4. Système (10) d'aspiration selon la revendication 1, dans lequel l'extrémité distale (32) du tube canulé (24) est une première extrémité évasée (28) et l'extrémité proximale (34) du tube canulé (24) est une deuxième extrémité évasée (30).

5. Système (10) d'aspiration selon la revendication 4, dans lequel un diamètre intérieur du tube canulé (24) s'accroît depuis une position centrale (36) vers la première extrémité évasée (28) et vers la deuxième extrémité évasée (30).

6. Système (10) d'aspiration selon la revendication 1, dans lequel l'extrémité distale (40) du premier adaptateur (38) a un quatrième diamètre (d4) qui est plus grand que le premier diamètre (d1) de l'embout distal (16) du dispositif (12) d'aspiration de Yankauer.

7. Système (10) d'aspiration selon la revendication 6, dans lequel l'extrémité proximale (46) du premier adaptateur (38) a un cinquième diamètre (d5) qui est plus grand que le premier diamètre (d1) de l'embout distal (16) du dispositif (12) d'aspiration de Yankauer.

8. Système (10) d'aspiration selon la revendication 7, dans lequel le cinquième diamètre (d5) est plus grand que le quatrième diamètre (d4).

9. Système (10) d'aspiration selon la revendication 1, comprenant en outre un ou plusieurs méplats (48) s'étendant le long de la longueur de l'extrémité proximale (46) du premier adaptateur (38).

10. Système (10) d'aspiration selon la revendication 1, dans lequel

l'extrémité distale (40) du premier adaptateur (38) inclut des barbelures extérieures (42) et l'extrémité proximale (20) du dispositif (12) d'aspiration de Yankauer a des barbelures extérieures (22), et

les barbelures extérieures (42) du premier adaptateur (38) se couplent avec les barbelures extérieures (22) sur l'extrémité proximale (20) du dispositif (12) d'aspiration de Yankauer.

11. Système (10) d'aspiration selon la revendication 1, comprenant en outre :

un deuxième instrument (12') ayant une extrémité proximale (20') configurée pour se coupler avec une extrémité distale (46') d'un deuxième adaptateur (38'), le deuxième adaptateur (38') ayant une extrémité proximale (42') configurée pour se coupler avec l'extrémité distale (32) du tube canulé (24) ;

dans lequel le dispositif (12) d'aspiration de Yankauer et le deuxième instrument (12') sont interchangeables de sorte que, dans une première configuration, l'extrémité proximale (20) du dispositif (12) d'aspiration de Yankauer est connectée à l'extrémité distale (32) du tube canulé (24), et dans une deuxième configuration, l'extrémité proximale (42') du deuxième instrument (12') est connectée à l'extrémité distale (46') du deuxième adaptateur (38'), qui est connectée à l'extrémité distale (32) du tube canulé (24).

12. Système (10) d'aspiration selon la revendication 11, dans lequel le deuxième instrument (12') est un dispositif d'aspiration de Yankauer à petit alésage qui a un diamètre intérieur plus petit que le diamètre intérieur (d1) du dispositif (12) d'aspiration de Yankauer.

13. Système (10) d'aspiration selon la revendication 11, dans lequel, dans la première configuration, le sens d'aspiration (f) est depuis l'embout distal (16) du dispositif (12) d'aspiration de Yankauer jusqu'à l'extrémité proximale (46) du premier adaptateur (38).

14. Système (10) d'aspiration selon la revendication 11, dans lequel, dans la deuxième configuration, le sens d'aspiration (f) est depuis le deuxième instrument (12') jusqu'à l'extrémité proximale (46) du premier adaptateur (38).

**15.** Système (10) d'aspiration selon la revendication 11, dans lequel

l'extrémité distale (40) du premier adaptateur (38), l'extrémité distale (46') du deuxième adaptateur (38'), et l'extrémité proximale (20) du dispositif (12) d'aspiration de Yankauer incluent chacune des barbelures extérieures (22, 42), et les barbelures extérieures (42) du premier adaptateur (38) se couplent sur les barbelures extérieures sur le deuxième adaptateur (38') et les barbelures extérieures (22) sur l'extrémité proximale (20) du dispositif (12) d'aspiration de Yankauer.

FIG. 1

10

FIG. 2

FIG. 3

FIG. 4

FIG. 5

FIG. 6

SECTION A—A

SECTION G—G

DETAIL A

FIG. 7

EP 3 765 107 B1

50

52

38

FIG. 8

**EP 3 765 107 B1**

REFERENCES CITED IN THE DESCRIPTION

**Patent documents cited in the description**

- US 5921970 A
- US 5665080 A
- US 4455140 A